# EUROPEAN PATENT SPECIFICATION

(11) **EP 0 658 195 B1**
(45) Date of publication and mention of the grant of the patent: **21.05.1997**
(21) Application number: 93919173.0
(22) Date of filing: 25.08.1993
(51) Int. Cl.: C12N 7/00, A61K 39/12, C12P 21/08, C07K 14/08, G01N 33/569, C12N 15/40

(54) **VIRUS OBTAINABLE FROM HUMAN NEUROBLASTOMA CELLS**
VON MENSCHLICHEN NEUROBLASTOMAZELLEN ERREICHBARES VIRUS
VIRUS OBTENU A PARTIR DE CELLULES HUMAINES DE NEUROBLASTOME

(30) Priority: 26.08.1992 GB 9218164
(43) Date of publication of application: 21.06.1995
(73) Proprietor: Applied Research Systems ARS Holding N.V., Curaçao (AN); ROVIGATTI, Ugo, I-00135 Rome (IT)
(72) Inventor: ROVIGATTI, Ugo, I-00135 Rome (IT)
(74) Representative: Woodman, Derek
(86) International application number: EP9302291
(87) International publication number: WO9404661

(56) References cited:
- PROC. AM. ASSOC. CANCER. RES. ANNU. MEET. vol. 32 , 1991 page 311 U. ROVIGATTI 'Presence of a new virus (MFV) associated with H-MYCN rearrangements in neuroblastoma cells in a cluster case'
- C. R. ACAD. SCI. SER III vol. 315, no. 5 , 1992 pages 195 - 202 U. ROVIGATTI 'Isolation and initial characterisation of a new virus: micro-foci inducing virus or MFV'

## Description

This invention relates to a virus and substances related thereto, in particular a virus isolated from human neuroblastoma cells, which virus induces small foci (micro-foci) in certain cell cultures and will be referred to herein as micro-foci inducing virus or MFV.

Neuroblastoma is believed to originate from the malignant transformation of neural crest progenitor cells which develop inter alia into the sympathetic nervous system, adrenal medulla and other tissues. Neuroblastoma can be classified into two distinct groups; the first is generally diagnosed at a younger age and has the better prognosis while the other group having the worst prognosis is accompanied by the appearance of specific markers which include increased levels of urinary catecholamines and of serum ferritin and genetic markers peculiar to tumour cells. Examples of such genetic markers in the more aggressive neuroblastoma type (second type) are: i) presence of a diploid karyotype (the first type of neuroblastoma appears to be aneuploid or triploid) ii) specific chromosomal deletions, particularly on chromosome 1p and iii) amplification of the proto-oncogene N-myc (in one case about 1,000 times). The amplification of N-myc appears to be an independent marker of poor prognosis.

Earlier studies of neuroblastoma have been inconclusive as to the exact nature of its cause. Reference has been made for example to a DNA virus (e.g. cytomegalovirus) being the causative agent. Others have suggested that neuroblastoma which arises in the neonate or very young child is a consequence of exposure in utero to a carcinogen. It has been observed that the majority of cases in a cluster of neuroblastoma cases showed birth related problems and there was a higher number of congenital malformations (particularly spina bifida) observed among children born in the same area and time period as the cases in the neuroblastoma cluster. Surprisingly, it has now been discovered that a virus comprising segmented, double-stranded RNA and having a particle diameter of about 72nm is present in neuroblastoma cells studied and that its presence causes N-myc amplification in infected cells.

According to one aspect of the invention, there is provided a virus of icosahedral structure having a diameter of about 72nm, double-stranded RNA in seven segments of molecular weights 4.0, 3.9, 2.3, 2.2, 1.6, 1.4 and 1.3 kb, which is capable of infecting human neural crest cells and other human ectodermal cells, causes N-myc amplification in infected cells and is obtainable from human neuroblastoma cells.

MFV particles can be found in large numbers in both the cytoplasm of infected cells as well as in the supernatants of culture media. MFV may be isolated from other substances derived from cells, e.g. neuroblastoma cells or other MFV-infected cells, by for example density gradient centrifugation of culture media supernatants. However, since most MFV will be in association with cytoplasm from infected cells it is preferable to first carefully homogenise cells, e.g. freeze/thaw three times in a dry ice/ethanol bath followed douncer homogenisation. MFV particles released by the above procedure are then advantageously concentrated by centrifugation through a density gradient, e.g. sucrose or cesium chloride or more preferably potassium tartrate. Such methods of isolating viral particles form an aspect of this invention. Isolated MFV particles which are free from cellular contaminants also form part of this invention. Such isolated particles may be lyophilised if desired and stored. Care has to exercised at all stages since the genetic material of MFV is very heat labile.

MFV can infect and transform other human cell types and cells from other animals, e.g. rats. Cell lines NBAT-14, NBAT-21 (Gamby, C et al Int. J. Cancer (in press), CHP-100 (Children's Hospital of Philadelphia, USA) and SK-N-SH (ATCC, Rockville, MD, USA) have been infected with MFV. Infected cell line designated MFV/SK-N-SH was deposited under the Budapest Treaty at the European Collection of Animal Cell Cultures, Porton Down, England on 25 August 1992 and has been assigned the deposit number V92082509. This deposited cell line also form part of this invention.

The RNA of MFV may be extracted from MFV particles by using any method which does not degrade the RNA. A known method is the solvent extraction method based on guanidium thiocyanate (see for example "Molecular Cloning" a laboratory manual, by J. Sambrook, E. Fritsch and T. Maniatis, Cold Spring Harbour 1989 or McGookin, R. in "Methods in Molecular Biology Vol.2, Humana Press (1984) Ed. J.M. Walker). The extracted RNA may be separated into the individual segments by, for example gel electrophoresis, it being recognised that longer gels and running times are required to separate the larger 4.0 and 3.9 kb bands and the 2.3 and 2.2 kb bands from each other. Each individual segment together with fragments thereof also form an aspect of this invention; the term fragment being intended to encompass portions large enough to serve as probes or larger e.g. at least 10 bases long.

Techniques are well known in the art for forming homologous DNA sequences using RNA templates and reverse transcriptase. Such techniques may be used with advantage to produce the more chemically and heat stable DNA homologues of the segments and fragments mentioned above. Such DNA homologues also form part of this invention.

Analysis of the sequences of the RNA segments will provide interesting information and comparison with known data will assist in, for example, the development of DNA probes for use as markers and in analysis of samples.

It has already been observed both in vivo and in vitro that there is clear genetic variation between MFV isolates or strains and this invention extends to non-deposited strains of MFV as well as attenuated and non-pathogenic strains developed by, for example, repeated passaging, U.V. irradiation and other techniques known to the skilled person.

It is desirable that simple and rapid tests be developed for the detection of MFV and antibodies against MFV. Also, it is desirable that a vaccine be produced to offer prophylaxis against MFV infection. To these ends, it is an aspect of this invention to provide attenuated, non-pathogenic or inactivated MFV particles or protein sub-components thereof as antigens.

In order to elicit antibody formation in subjects, the antigens, either alone or linked to a carrier molecule (i.e. covalently bonded or physically absorbed on a carrier by known methods in order to optimise their immunological function), may be administered by any route (eg parenteral, nasal, oral, rectal, intravaginal), with or without the use of conventional adjuvants (such as aluminium hydroxide or, in the case of laboratory animals, Freund's complete or incomplete adjuvants) and/or other immunopotentiating agents. The invention also includes formulation of antigens according to the invention in slow-release forms, such as a sub-dermal implant or depot comprising, for example, liposomes (Allison, A.C. & Gregoriadis, G. (1974) Nature (London) 252, 252) or biodegradable microcapsules manufactured from co-polymers of lactic acid and glycolic acids (Gresser, J. D. and Sanderson, J. E. (1984) in "Biopolymer Controlled Release Systems" pp 127-138, Ed. D. L. Wise).

Antigens according to the invention may be used either alone or linked to an appropriate carrier, as:
(a) vaccines, for use to prevent infection by MFV;
(b) ligands in assays of, for example, samples such as serum from positive or suspected subjects;
(c) antigens for in vitro cellular bioassays e.g. the gamma-interferon assay which detects gamma-interferon released in response to specific antigen in a whole blood culture;
(d) quality control agents in testing, for example, binding levels of antibodies raised against the antigens; and
(e) immunogenic agents for the generation of monoclonal or polyclonal antibodies by immunisation of an appropriate animal, such antibodies being of use for (i) the scientific study of the MFV, and (ii) as diagnostic agents, e.g. as part of histochemical reagents. The invention further provides for genetically engineered forms or sub-components, especially V_{H} regions, of antibodies raised against the antigens, and of humanised forms of antibodies initially raised against the antigens in other animals, using techniques described in the literature.

In respect of detection and diagnosis of MFV or antibodies against MFV, the skilled person will be aware of a variety of immunoassay techniques known in the art, inter alia, sandwich assay, competitive and non-competitive assays and the use of direct and indirect labelling. In several assays techniques, labelled antigen may be used and the type of label will be dependent on many factors which are recognised by the skilled person. It will be appreciated that labelled antigen is another aspect of this invention.

A further aspect of the invention provides a kit for detecting MFV or antibodies against MFV which comprises at least one antigen according to the invention.

The preparation of polyclonal or monoclonal antibodies, recombinant forms particularly adapted to the species of interest, e.g. humanised forms of antibodies (see, for example, Thompson K. M. et al (1986) Immunology 58, 157-160), single domain antibodies (see, for example, Ward, E. S., Gussow, D., Griffiths, A. D., Jones, P. and Winter, G. (1989) Nature 341, 544-546), which bind specifically to an antigen according to the present invention, may be carried out by conventional means and such antibodies are considered to form part of this invention.

It will be appreciated that diagnostics kits according to the invention can be used not only to determine possible infection but also, in the case of pregnancy to assess potential teratogenic effects in unborn fetuses. As mentioned above, a link has been observed between the incidence of neuroblastoma, birth-related problems and a high incidence of congenital malformation (particularly spina bifida).

Antibodies according to the invention are, inter alia, of use in a method of diagnosing MFV infection in a subject which comprises incubating a sample of tissue or body fluid of said subject with an effective amount of antibody as described herein and determining whether, and if desired the extent to which and/or rate at which, cross-reaction between said sample and said antibody occurs. Diagnostic kits which contain at least one of said antibodies also form part of this invention. It will be understood that the antibodies raised against at least one antigen according to the invention may be labelled for use in, for example, diagnostic kits and histochemical reagents and that such labelled antibodies also constitute an aspect of the invention.

Antibodies raised by immunisation using an antigen according to the invention can be used to raise anti-idiotypic antibodies which also form part of this invention.

A further aspect of the invention provides antigens as defined above for use in therapy or prophylaxis of MFV infection in a subject and/or for stimulating the immune system of a subject and for the preparation of medicaments suitable for such uses. Also included are pharmaceutical compositions containing, as active ingredient, at least one antigen or antigen-carrier conjugate as described herein in association with one or more pharmaceutically acceptable adjuvants, carriers and/or excipients. The compositions may be formulated for oral, rectal, nasal or especially parenteral administration.

The invention further provides a method of therapy or prophylaxis of MFV infection and/or of stimulating the immune system, which comprises administering an effective amount of an antigen as hereinbefore defined to a subject.

The invention will now be described by way of a non-limiting example.

Between the years 1986 and 1989 an excessive number of neuroblastoma cases were diagnosed in an area of Southern Louisiana. Initially, an excessive rate of new cases was detected in the relatively small city of Morgan Town in Southern Louisiana but during the same period, however, several paediatric oncology groups throughout the New Orleans area observed an unusually large number of neuroblastoma cases, (ten times the national average).

In one case a large kidney tumor was removed from a three year old girl and Southern blotting analysis disclosed the presence of the N-myc proto-oncogene highly amplified (approximately 1000 times). The same tumor cells, after being placed in tissue culture, displayed an extremely high variation of N-myc copy number, which correlated with the presence of small foci of rounded cells which were clearly distinguishable from the monolayer of large mesenchymal cells; the majority cell of the primary cultures. It was found that the appearance of such small foci (called Micro-Foci) could be induced in cultures containing only monolayers of large mesenchymal cells by infection with ultrafiltered supernatants of Micro-Foci containing cultures. The inducing agent could be passaged as an ultrafilterable infectious agent and was therefore called Micro-Foci inducing Virus or MFV. FHs-173 WE human embryonic cells were infected with ultrafiltered supernatants and showed some cytopathic effect accompanied by Micro-Foci formation. It was found that MFV-transformed FHs-173 WE cells grown in 2% fetal calf serum (FCS) with a division time of approximately 20 hours (compared to 36 hours for uninfected cells) make colonies in semisolid medium (0.34% agarose) with an efficiency of plating (EOP) of 16%, while uninfected cells do not grow. A large number of virus particles were detected by electron microscope (EM) in ultrafiltered supernatants, in the original cultures and in infected FHs-173 WE cells. Higher magnification EM analysis revealed an icosahedral structure with a diameter of approximately 72 nanometers. Further EM studies have shown that virus particles visualized by negative and positive staining and corresponding to MFV are 1) present in the ultrafiltered supernatants of the original neuroblastoma cultures and in cultures infected with such ultrafiltered supernatants; 2) present inside the cytoplasm of neuroblastoma cultures and in human cells infected by ultrafiltered supernatants; 3) present inside cells (and in ultrafiltered supernatants obtained from such cells) of newborn animals in which injection of MFV in their parents induced a very specific and lethal syndrome resembling neuroblastoma (see below).

Homology between the MFV genome and other human and non-human viruses was sought by hybridization with probes specific for HSV-1, CMV, JC virus, BK virus Vaccinia Virus and SV40 (obtained from ATCC, Rockville, MD, USA); HIV and Polio (obtained from Univ. Mass. Med. School, MA, USA) and, EBV (obtained from Linenberger Can. Res., NC, USA) which gave negative results. Partially positive hybridization was obtained employing a probe specific for the early region genes of an oncogenic Adenoviridiae (Adenovirus-12 obtained from the Cancer Center Mass. Gen. Hos., MA, USA). A similar hybridization pattern was obtained from DNA of the original tumor (thus excluding any possibility of contamination in culture), from DNA of neuroblastoma cultures and DNA from transformed cells of affected animals. In animal experiments, a total of eighteen litters were obtained from rats of Fisher 344 strain: 7 from uninfected control animals and 11 from infected parents which had been given sterile syringe injections with ultrafiltered supernatants in the abdominal cavity. All the infected litters developed between two and four weeks of age a syndrome characterised by ataxia, seizures, extensive cyanosis, particularly in the head, tail and paws, watery diarrhoea for several days and death. Nodules of small, round and blue cells which appeared transformed when grown in culture were present under the skin of dying animals and in internal organs. None of the animals from control litters showed any of these symptoms, while all of the 11 litters from infected parents of the strain Fisher 344 showed these symptoms, fatal in the majority of cases.

Cells obtained from the affected newborns displayed transformed properties in vitro, presence of MFV by infectivity and EM studies and cross-hybridisation with the MFV-hybridising probe. Further studies have shown that human neuroectodermal cells containing a normal diploid content of N-myc can be infected and transformed by MFV. Preliminary results employing DNA extracted from two such neuroectodermal cell lines normally with diploid N-myc content (SK-N-AS and VA-N-BR) before infection and four passages after MFV infection show that the amount of N-myc amplification in MFV-infected cells is 50-100 fold.

### 1. PURIFICATION OF MFV

MFV has been purified from an impure source of the virus employing several different methods. One method has been optimised and allows purification (up to 5000 times) of large amounts of MFV in relatively short periods of time. Supernatants from neuroblastoma cultures or from MFV-infected human cells may be used as source materials.

### Preparation of cell culture media.

All media are prepared employing triple-distilled water. Different modifications of Eagle's minimal essential medium (MEM) are employed. For cultures growing as monolayers (the majority of cultures for MFV assays and production) a 50% mixture of Dulbecco's modified MEM and McKoy's medium were employed in the presence of 10% Fetal Calf Serum (FCS, LifeScience; Gibco-BRL, Maryland). It was observed that it is extremely important to screen and select FCS batches in order to obtain optimal results. Antibiotics are added as 100 U of penicillin G (Sigma) per liter and 20 ug of streptomycin sulfate (Sigma) per liter. Alternatively, 50 ug of Gentamycin/liter (Schering Co.) were utilised. Trypsin-EDTA solution were obtained from LifeSience (MD, USA), while stock solutions of Phosphate-Buffered Saline (PBS) were obtained from Sigma (St. Louis, MO, USA).

### Infection of cultured cells.

Typical cultured cells, such as the NBAT-14 or NBAT-21 cell lines described later, are collected by centrifugation at approximately 200 g for 15 seconds (after trypsinization and neutralization of trypsin with 10% FCS containing medium). Cells are infected with 20-100 MF inducing units of MFV in the presence of medium without serum and then left to attach in the same medium to the plastic surface of the culture vessel (the absence of serum will speed up adhesion of the cells, unpublished results). DEAE-Dextran at a concentration of 20 ug/ml is also employed in this first phase in order to increase the efficiency. Normal medium is then added and the cells are allowed to grow at 37°C for 10-14 days before being harvested for virus purification.

### Centrifugation of MFV.

MFV is concentrated from the supernatants of infected cells by centrifugation at 10000 rpm (Beckman or Sorval centrifuge). The whitish viral pellet is then resuspended and processed together with the material extracted directly from cells. Since most of MFV will be present in association with the infected cytoplasm particular care must be taken during cellular homogenization. Cells are frozen/thawed three times in a dry-ice/ethanol bath and then further treated by sonication and douncer homogenization. After the first thawing all procedures must be carried out at 0-4°C (this is due to extreme lability of MFV genetic material, as illustrated in the following Section). Released MFV particles are again concentrated at 10000 rpm. Centrifugation through 10-40% sucrose gradient will allow a better purification through sedimentation gradients. Cesium chloride gradients between 1.2 and 1.4 g/ml allow a good purification of an MFV band at approximately 1.33 g/ml. Excellent results were also obtained by purifying MFV through 10-50% potassium tartrate gradient: the virus bands in the lower portion of the gradient with empty particles being visible in a slightly higher position.

### 2. STABILITY OF THE MFV GENOME AND ITS DETECTION

One puzzling phenomenon in trying to understand how MFV infects human cells, which could be associated with genetic changes, has been our incapability to detect its genome and to understand its structure. This uncertainty was rendered even more confusing in view of the other evidence for MFV presence in human cells in cultures as well as in affected animals (electron microscope data). Moreover, when studying nucleic acid extracts from affected cells (which extracts contained both DNA and any RNA not degraded by the extraction procedure) there was also the unusual appearance of bands in many gels run with the specific intention of separating genomic DNA fragments, e.g. Southern blotting assays after restriction enzyme digestion. These bands were clearly visible after ethidium bromide staining and typically present in cells derived from the original nuroblastoma tumor which exhibited extremely high N-myc amplification.

Since the bands appeared in ethidium bromide stained gels for Southern Blotting, one possible interpretation was that such bands derived from staining of repetitive DNA (for example satellite DNA) after digestion with a particular restriction enzyme (for example, Eco-R1). Data against such a hypothesis were, however, the fact that the intensity of the bands could easily vary among cells from the same neuroblastoma culture and - more importantly - the fact that one culture could be "converted" to show the presence of such bands. Initial experiments trying to closely associate the presence of such bands with MFV infection were hampered by the fact that, again, their presence and intensity showed great variation.

Finally, it was observed that several ectodermal lines obtained from ectodermal tissue of breast benign lesions were infected and efficiently transformed by MFV. One of such line NBAT-14 is particularly resistant to cytopathic effects induced by the virus and therefore provided an ideal vehicle for its propagation and study. It was observed that different Southern blotting gels with nucleic acid from MFV-transformed NBAT-14 cells would show a different ethidium bromide banding pattern. Eventually, the same nucleic acid preparation showed two completely different banding patterns after an interval of few hours. It was then realized that these bands are extremely heath labile, thus explaining why they can suddenly disappear (it should be kept in mind that the majority of the restriction enzyme digestions for Southern blotting are incubated at 37°C, usually for at least one hour). In one control experiment in which the same nucleic acid preparation was kept at room temperature for variable amounts of time, it was finally verified that the suspect banding pattern (probably associated with MFV) could be completely destroyed just by incubating the sample at room temperature for five minutes (without addition of any restriction enzyme or incubation buffer).

The identity of such banding pattern with the genome of MFV was obtained by analyzing a larger number of human cell lines (both of neuroectodermal and ectodermal origin) after infection and transformation with MFV. Only the nucleic acids extracted from the infected lines contained the above-described banding pattern, detected by ethidium bromide staining. Nucleic acids extracted from the uninfected NBAT-14 cells contain only high molecular weight DNA and a small amount of RNA while nucleic acids extracted from MFV-transformed NBAT-14 cells also contain the above mentioned specific bands. Interestingly, the MFV specific bands can be extracted from a gel and rerun with the same banding appearance. The RNA pattern after ethidium bromide staining after extraction from ten different cell lines infected by MFV also shows the banding pattern clearly and the banding is completely consistent among the different nucleic acid preparations. In this respect, it should be noted that some clear genetic variation for MFV has been observed both in vitro and in vivo (animal model, unpublished observations). Although the most extensively studied isolate of MFV has been deposited as described above, this invention also relates to genetically related isolates, which may be identified by different genome size bands.

### 3. NATURE OF MFV NUCLEIC ACIDS

The results described in Section II indicate that the ethidium bromide staining bands which appeared on gels for Southern blotting assays were not the result of the presence of repetitive DNA sequences in the genome of most eukaryotic species. Indeed, repetitive DNA sequences in the genome would show a banding pattern after ethidium bromide staining only as a consequence of digestion with a particular restriction enzyme which would always introduce cuts at the same site, thereby generating the repetitive pattern. This hypothesis was proved completely wrong by the experiments described in Section II. Those experiments clearly indicated that such a banding pattern was not created by restriction enzyme digestion and that such digestion can in fact destroy the characteristic banding pattern.

The bands shown after ethidium bromide staining are the "bona fide" genome of MFV since 1) they appear only in MFV infected cells and 2) their pattern is very reproducible. The segmented nature of the MFV genome opens a question about the nucleic acids themselves, since it would be unusual to detect several DNA molecules with such a specific banding pattern. The hypothesis of a virus with a segmented RNA genome appeared to be more plausible and was tested.

Additional evidence in this respect of a segmented RNA genome was obtained:
1) by digesting the total nucleic acid preparation with higher concentration of RNase A (200 ug/ml; Sigma, St. Louis, MO, USA), which destroys the segmented pattern of MFV. Indeed the RNAase concentration employed in standard DNA extraction in the laboratory were too low to digest the MFV RNA (most likely double stranded, i.e. 10 ug/ml);
2) by digesting the total nucleic acid preparation with DNase (approximately 250 ug/ml; 500 Kunitz units (Sigma)) which does not appear to significantly affect the segmented MFV genome;
3) by staining the extracted nucleic acids with different dyes specific for single and double stranded nucleic acids (such as ethidium bromide) or with higher affinity for single stranded RNA molecules (such as acridine orange).

Interestingly, the dye 4',6-diamine-2-phenylindole dihydrochloride (DAPI; Boehringer Mannheim, Mannheim W. Germany) shows the peculiar property of binding only double stranded nucleic acid (both DNA and RNA). Employing this stain, it was found that the segmented MFV genome can be clearly and intensely stained by DAPI, while ssRNA is not. Furthermore, the MFV segments stained by DAPI have a peculiar "greenish" fluorescence typical of dsRNA staining. However, probably the strongest evidence in favour of an RNA genome for MFV comes from selective extractions of nucleic acids.

Four aliquots containing same number of cells (i.e. 10⁷) were extracted by different methods. Two infected lines (MFV-NBAT-14) and one uninfected control (NBAT-14) were extracted with classical detergent methods for DNA (phenol/chloroform extraction, see e.g. Molecular Cloning or Methods in Molecular Biology, ibid). The fourth sample was the same infected line extracted by a solvent method selective for RNA molecules (based upon guanidnium thiocyanate). Even a five-fold dilution of the latter extraction gave the characteristic banding. It is clear from this experiment that the yield of segmented MFV genome is much higher (at least ten times) in the RNA enriched fraction and this confirms the RNA nature of the MFV genome.

### 4. CLONING OF MFV PORTIONS

The detection of the MFV genome and its identification as an RNA molecule with segmented nature has allowed its cloning, by cDNA synthesis (essentially according to the method of Gubler, U. and Hoffman, B.J., Gene 25: 263-269 (1983) and ligation into a plasmid vector.

Initially, the genome was divided into three different groups of bands with similar molecular weights. Although one can immediately distinguish five different bands, two additional bands can be separated in both the high and the intermediate molecular weight (MW) ranges by running the nucleic acid in longer gels (agarose 7%) for longer times (more than one day). A total of seven bands or segments accounts for the MFV genome with molecular weights of 4.0, 3.9, 2.3, 2.2, 1.6, 1.4 and 1.3 kb. These segments are grouped in three different groups of "high M.W." (including the 4.0 and 3.9 kb bands), "intermediate M.W." (including the 2.3 and 2.2 kb bands), "low M.W." (including the 1.6, 1.4 and 1.3 kb bands). Such bands and groups of bands can be easily extracted from a gel and rerun after extraction in another gel where they maintain their original nature and identity.

The strategy of cloning adopted is characterised by:
1) cloning of the three different M.W. groups individually, but without further separating individual molecular clones till the actual clones were obtained: this allows considerable simplification of the procedure by producing three types only of cDNA molecules (the three M.W. groups). All the additional steps were also performed on the three different products only;
2) using a random primer (p-dN-6) for the initiation of cDNA synthesis by the AMV reverse transcriptase (Boehringer Mannheim, Mannheim, W. Germany);
3) employing the RNA-DNA hybrid as the actual substrate for the synthesis of the second DNA strand: this was done by milder treatment with RNAase-H (Boehringer Mannheim, Mannheim, W. Germany) which essentially produces only nicks in the RNA strand thus allowing the same molecule to provide the required 3'-OH priming moiety for DNA polymerase 1;
4) Using Kornberg's enzyme which is known to contain 5'→3' nuclease activity which can therefore remove the RNA primers in the direction of synthesis; it should be stressed that this method efficiently eliminates the problem often generated by hairpin structures which are typically created in other cDNA cloning strategies; and
5) Using T4 DNA polymerase which efficiently removes overhanging 3'-ends and therefore generates molecules with blunt ends for further DNA ligation into plasmid vectors.

The whole procedure briefly summarised here is a standard cDNA cloning strategy and is clearly described by Gubler and Hoffmann (ibid) and elsewhere, e.g. Molecular Cloning (ibid). A more detailed scheme of the cloning steps followed in producing the initial MFV clones is presented below. Most of the reagents employed in this cDNA cloning were obtained from Boehringer Mannheim, Mannheim, W. Germany.
1. A mixture of RNase inhibitor, deoxynucleotide cocktail, first DNA strand buffer, random primer p-dN-6, redistilled water, P³² labelled nucleotides, MFV purified RNA (divided, as previously described,into three different M.W. groups; in other words three different reactions plus one additional control reaction are run in parallel); and reverse transcriptase from Avian Myeloblastosis Virus (AMV) were incubated at 42°C for 60 minutes.
2. The mixture was then placed at 0-4°C and all the components for the second strand synthesis were added. They include: the buffer for the second DNA strand synthesis, RNase-H enzyme; redistilled water; P³² labelled nucleotides and Kornberg's enzyme (DNA polymerase 1). Incubation for this stage was; one hour at 12°C, one hour at 22°C and 10 minutes at 65°C.
3. To the same reaction mixture, the T4 DNA polymerase was added and the tube was left incubating for 10 minutes at 37°C.
4. The reaction was stopped by addition of a solution containing 0.2M EDTA(pH 7.2) and 10% Sarkosyl.
5. Before cloning, the products of the cDNA molecules were extracted with phenol/chloroform and precipitated with ethanol.
6. The resuspended cDNA products were ligated into appropriately prepared fragments of plasmid vectors. Plasmid vectors pBR-322 and pUC-19 were used, employing a strategy already successively utilised in cloning different oncogene portions of the avian virus MH2. For this purpose the blunt ended cDNA molecules were first ligated to a 10mer Eco-R1 linker (Promega, Madison, Wisconsin, USA), digested with the same enzyme and finally ligated to the appropriate vector fragments following the strategy described in "Transfection analysis of the oncogenes present in MH 2 Virus", the 27th report in: "Summary of Research, Site Visit 1985", Division of Cancer Etiology, National Cancer Institute, USA.

### 5. ADDITIONAL EFFECTS OF MFV UPON HUMAN CELLS

As indicated earlier, preliminary data had shown that neuroectodermal lines SK-N-AS and VA-N-BR, normally with a diploid N-myc content display a dramatic amplification of this proto-oncogene. These data have been now extended to other neuroectodermal lines discussed below.

The CHP-100 cell line provided by the Children's Hospital of Philadelphia, displays a typical neuroectodermal appearance in culture. Upon infection by MFV, CHP cells start showing (after a period of 10-14 days) a transformed phenotype. This is characterised by a more refractile cellular and particularly nuclear morphology, with nucleoli being particularly prominent. Cell line SK-N-SH was obtained from ATCC (Rockville, MD, USA). It normally shows also a neuroectodermal phenotype, but with more mesenchymal features. Approximately ten days after MFV infection, SK-N-SH cells start showing the presence of masses composed of much smaller cells, with rounded shapes and neural features. These cells closely resemble the cells present in the micro-foci described earlier. Both cells also display neural markers such as Neuron Specific Enolase (NSA, by immunohistochemistry). Additionally, obvious dendritic processes resembling neurites are observed which are similar to those observed in the original micro-foci.

Southern blotting analysis was performed on several cultures obtained from both CHP-100 and SK-N-SH cells after MFV infection and transformation. After digestion with the enzyme Eco-Rl and probing with plasmid pNb-1 insert (specific for N-myc), in comparison with normal control DNAs there is a dramatic amplification of this proto-oncogene in the MFV-infected and transformed cultures. In this case, it is also apparent that a rearranged band is present, in addition to the 2 kb "germ line" band (amplified). Amplification is present in MFV-infected/transformed SK-N-SH cells. The amount of N-myc amplification was estimated at approximately 20 fold and in the case of MFV-infected/transformed CHP100 cells approximately 50 fold.

While performing these experiments it was observed that the DNA extracted from MFV-infected/transformed cells (in which N-myc amplification was detectable) presented an excessive degradation of DNA (visible in the ethidium bromide staining of gels run for Southern blotting analysis). This pattern also correlated with morphological changes of the MFV-transformed cells after they reached confluence (in other words, when they kept growing on top of the monolayer, sometimes creating micro-foci or larger, floating masses). A majority of these cells appeared irregular in shape and "shrunken" and 50-85% of them could be stained by trypan blue. The effective "hyperdegradation" of the DNA from MFV-infected/transformed cells was clearly demonstrated when their DNA was run at 4°C without prior restriction enzyme digestion.

These data have been now confirmed in several independent experiments, that the presence of N-myc amplification induced by MFV in cells growing after confluence always correlates with DNA fragmentation. This phenomenon appears to be a specific example of apoptosis. In this respect, it is interesting to observe that a very recent report (Evan, G.I., et al Cell 69; 119-128 (1992) has also associated the presence of a highly expressed c-myc gene with the induction of apoptosis.

### 6. EXPRESSION OF RECOMBINANT MFV PROTEIN

The invention also extends to recombinant MFV and a method for its production.

A DNA insert comprising the MFV gene may thus be incorporated into a suitable vector which is then used to transform or transfect an appropriate host organism. Suitable methods are well known to those skilled in the art and may be found in textbooks such as Molecular Cloning (ibid) or Methods in Molecular Biology (ibid).

Although prokaryotic and yeast hosts may be used, it is preferred to use mammalian cells as hosts. It is thus preferable to use human or animal fibroblast or myeloma cells lines such as HeLa, a human cell line; BHK - baby hamster kidney cells; VERO, a monkey kidney cell line; FR 3T3, Fisher rat fibroblasts; CHO, a Chinese hamster ovary cell line or rat myeloma cell lines such as YB2/0 and Y3. CHO cell lines are particularly preferred.

Vectors appropriate for different classes of mammalian cell lines are well known in the art. In general, these will comprise a promoter and/or enhancer operably connected to a gene expressing the target protein. Suitable promoters include SV40 early or late promoter, eg. PSVL vector; cytomegalovirus (CMV) promoter; mouse melallothionein I promoter and mouse mammary tumour virus long terminal repeat.

Transfection of host cells may be effected using standard techniques, for example using calcium phosphate, DEAE-dextran, polybrene, protoplast fusion, liposomes, direct microinjection, gene cannon or electroporation. The latter technique is generally applicable and is described by Andreason GL and Evans GA, Biotechniques 6, 65, 1980). In general, linear DNA is more readily introduced than circular DNA.

Thus, for example the DNA insert from the cloned pBR-322 vector may be cleaved with EcoRI and transferred to an expression vector suitable for the chosen host organism. If necessary, linkers may be attached to the EcoRI-cleaved ends to permit ligation into the selected vector.

After the introduction of the vector, the host cells are grown in a selective medium, which selects the growth of vector-containing cells. Expression of the cloned gene sequence results in the production of the MFV protein, or a fusion protein, or a mutant or a fragment thereof, depending on the DNA insert which has been used to transform the appropriate host. The expressed protein is then isolated and purified by any conventional procedure involving extraction, precipitation, chromatography, electrophoresis, or the like, or by affinity chromatography using anti-MFV monoclonal antibodies immobilized on a gel matrix contained within a column.

## Claims

1. A virus of icosahedral structure having a diameter of about 72nm, double-stranded RNA in seven segments of molecular weights 4.0, 3.9, 2.3, 2.2, 1.6, 1.4 and 1.3 kb, which is capable of infecting human neural crest cells and other human ectodermal cells, causes N-myc amplification in infected cells and is obtainable from human neuroblastoma cells.

2. A virus as claimed in claim 1 as deposited at the European Collection of Animal Cell Cultures, Porton Down, in the transfected cell line V92082509.

3. A virus as claimed in claim 1 or claim 2 free from cell contaminants.

4. A cell line of human or animal origin transfected with a virus as claimed in claim 1.

5. The RNA of a virus as claimed in claim 1 or claim 2 and fragments thereof.

6. DNA corresponding to the RNA as claimed in claim 5.

7. Plasmid vectors containing DNA as claimed in claim 6.

8. An antigen comprising a virus as claimed in claim 1 or claim 2 in attenuated or inactivated form or a protein sub-fragment thereof.

9. A pharmaceutical composition comprising an antigen as claimed in claim 8, optionally conjugated to a carrier molecule, together with a pharmaceutical adjuvant, carrier and/or excipient.

10. Monoclonal or polyclonal antibodies against a virus or antigen as claimed in claim 1 or claim 8 or immunologically active fragments thereof.

11. A kit for detecting a virus as claimed in claim 1 or antibodies as claimed in claim 10 comprising at least one antigen as claimed in claim 8 and/or at least one antibody as claimed in claim 10.

12. Anti-idiotypic antibodies derived from antibodies as claimed in claim 10.

13. Use of an antigen as claimed in claim 8 in the manufacture of a composition for use in combatting microfocus inducing virus infection.

14. A method of isolation of a virus as claimed in claim 1 or claim 2 in which cells infected with said virus are lysed to release the virus particles and subjected to density gradient centrifugation to separate the virus particles from cell contaminants.

15. Recombinant protein of the virus as claimed in claim 1.

16. A method of producing recombinant protein as claimed in claim 15 wherein host cells are transformed or transfected with a vector containing DNA of the virus as claimed in claim 1 coding for said protein and cultivated in a selective medium for recovering said protein from the culture of said host cells.

## Revendications

1. Virus de structure icosaèdre ayant un ARN double-brin d'environ 72 nm de diamètre en sept segments ayant des poids moléculaires de 4,0, 3,9, 2,3, 2,2, 1,6, 1,4 et 1,3 kb, qui est capable d'infecter des cellules de crêtes neurales humaines et d'autres cellules ectodermiques humaines, provoque une amplification N-myc dans des cellules infectées et peut être obtenu à partir de cellules humaines de neuroblastomes.

2. Virus suivant la revendication 1, tel que déposé à l'European Collection of Animal Cell Cultures, Porton Down, dans la lignée cellulaire transfectée V92082509.

3. Virus suivant les revendications 1 ou 2, exempt de contaminants cellulaires.

4. Lignée cellulaire d'origine humaine ou animale transfectée par un virus suivant la revendication 1.

5. ARN d'un virus suivant les revendications 1 ou 2, et fragments de celui-ci.

6. ADN correspondant à l'ARN suivant la revendication 5.

7. Vecteurs plasmidiens contenant un ADN suivant la revendication 6.

8. Antigène comprenant un virus suivant les revendications 1 ou 2 sous une forme atténuée ou inactivée ou sous-fragment de protéine de celui-ci.

9. Composition pharmaceutique comprenant un antigène suivant la revendication 8, éventuellement conjugué avec une molécule de support, avec un adjuvant, un support et/ou un excipient pharmaceutiques.

10. Anticorps monoclonaux ou polyclonaux dirigés contre un virus ou un antigène suivant les revendications 1 ou 8 ou des fragments de ceux-ci actifs du point de vue immunologique.

11. Trousse pour la détection d'un virus suivant la revendication 1 ou des anticorps suivant la revendication 10, comprenant au moins un antigène suivant la revendication 8 et/ou au moins un anticorps suivant la revendication 10.

12. Anticorps anti-idiotypiques dérivés d'anticorps suivant la revendication 10.

13. Utilisation d'un antigène suivant la revendication 8 dans la fabrication d'une composition utile dans la lutte contre une infection par un virus induisant des microfoyers.

14. Procédé pour l'isolement d'un virus suivant les revendications 1 ou 2, dans lequel des cellules infectées par ce virus sont lysées pour libérer les particules de virus et soumises à une centrifugation dans un gradient de densité pour séparer les particules de virus des contaminants cellulaires.

15. Protéine recombinante du virus suivant la revendication 1.

16. Procédé pour la production d'une protéine recombinante suivant la revendication 15, dans lequel des cellules hôtes sont transformées ou transfectées par un vecteur contenant l'ADN du virus suivant la revendication 1 codant pour cette protéine et cultivées dans un milieu sélectif pour la récupération de cette protéine à partir de la culture de ces cellules hôtes.

## Patentansprüche

1. Virus mit icosaedrischer Struktur mit einem Durchmesser von etwa 72 nm, doppelsträngiger RNA in sieben Segmenten mit Molekulargewichten von 4,0, 3,9, 2,3, 2,2, 1,6, 1,4 und 1,3 kb, das die Zellen des menschlichen Neuralrohres oder anderer menschlicher ectodermaler Zellen infizieren kann, in den infizierten Zellen N-myc-Amplifikation verursacht und aus menschlichen Neuroblastomzellen erhältlich ist.

2. Virus nach Anspruch 1, hinterlegt bei der European Collection of Animal Cell Cultures, Porton Down, in der transfizierten Zellinie V92082509.

3. Virus nach Anspruch 1 oder 2 ohne Zellverunreinigungen.

4. Zellinie menschlichen oder tierischen Ursprungs, transfiziert mit einem Virus nach Anspruch 1.

5. RNA eines Virus nach Anspruch 1 oder 2 und Fragmente davon.

6. DNA entsprechend der RNA nach Anspruch 5.

7. Plasmidvektoren, enthaltend DNA nach Anspruch 6.

8. Antigen, umfassend ein Virus nach Anspruch 1 oder 2, in attenuierter oder inaktivierter Form oder ein Proteinsubfragment davon.

9. Pharmazeutische Zusammensetzung, umfassend ein Antigen nach Anspruch 8, ggf. konjugiert an ein Trägermolekül zusammen mit einem pharmazeutischen Adjuvans, Träger und/oder Exzipienz.

10. Monoclonale oder polyclonale Antikörper gegen ein Virus oder Antigen nach Anspruch 1 oder 8 oder immunologisch aktive Fragmente davon.

11. Kit zum Nachweis eines Virus nach Anspruch 1 oder von Antikörpern nach Anspruch 10, umfassend mindestens ein Antigen nach Anspruch 8 und/oder mindestens einen Antikörper nach Anspruch 10.

12. Antiidiotypische Antikörper, abgeleitet von den Antikörpern nach Anspruch 10.

13. Verwendung eines Antigens nach Anspruch 8 zur Herstellung einer Zusammensetzung zur Verwendung zur Bekämpfung einer Mikrofocus-induzierenden Virusinfektion.

14. Verfahren zur Isolierung eines Virus nach Anspruch 1 oder 2, wobei mit dem Virus infizierte Zellen unter Freisetzung der Virusteilchen lysiert werden und einer Dichtegradientenzentrifugation zur Abtrennung der Virusteilchen von den Zellverunreinigungen unterworfen werden.

15. Rekombinantes Protein des Virus nach Anspruch 1.

16. Verfahren zur Produktion eines rekombinanten Proteins nach Anspruch 15, wobei die Wirtszellen mit einem Vektor transformiert oder transfiziert werden, der DNA des Virus nach Anspruch 1 enthält, die das Protein codiert und in einem Selektivmedium zur Isolierung des Proteins aus der Kultur der Wirtszellen gezüchtet werden.
